(19) Europäisches Patentamt / European Patent Office / Office européen des brevets

(11) **EP 2 836 121 B1**

(12) **EUROPEAN PATENT SPECIFICATION**

(45) Date of publication and mention
of the grant of the patent:
**26.10.2016 Bulletin 2016/43**

(21) Application number: **13717839.8**

(22) Date of filing: **12.04.2013**

(51) Int Cl.:
*A61B 5/145* (2006.01)    *A61B 5/048* (2006.01)

(86) International application number:
**PCT/GB2013/050948**

(87) International publication number:
**WO 2013/153398 (17.10.2013 Gazette 2013/42)**

(54) **BLOOD GAS DETERMINATION**

BLUTGASBESTIMMUNG

DÉTERMINATION DES GAZ DU SANG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.04.2012 GB 201206595
26.09.2012 GB 201217192
12.11.2012 GB 201220342**

(43) Date of publication of application:
**18.02.2015 Bulletin 2015/08**

(73) Proprietor: **The University Of Manchester
Manchester M13 9PL (GB)**

(72) Inventors:
• **VICTOR, Suresh
Manchester M13 9WL (GB)**
• **GAYDECKI, Patrick
Manchester M13 9PL (GB)**
• **MCKEERING, Christopher
Manchester M13 9WL (GB)**

(74) Representative: **Benson, Christopher et al
HGF Limited
4th Floor, Merchant Exchange
17-19 Whitworth Street West
Manchester M1 5WG (GB)**

(56) References cited:
• WIKSTROM SVERRE ET AL: "Carbon Dioxide and Glucose Affect Electrocortical Background in Extremely Preterm Infants", PEDIATRICS, vol. 127, no. 4, April 2011 (2011-04), pages E1028-E1034, XP8163580,
• VICTOR SURESH ET AL: "Effect of carbon dioxide on background cerebral electrical activity and fractional oxygen extraction in very low birth weight infants just after birth", PEDIATRIC RESEARCH, vol. 58, no. 3, September 2005 (2005-09), pages 579-585, XP8163579, ISSN: 0031-3998 cited in the application
• RAINA A ET AL: "EFFECT OF CARBON DIOXIDE ON BACKGROUND CEREBRAL ELECTRICAL ACTIVITY IN PRETERM INFANTS DURING THE FIRST 4 WEEKS AFTER BIRTH", PEDIATRIC RESEARCH, vol. 68, no. Suppl. 1, November 2010 (2010-11), page 56, XP8163582, & 3RD CONGRESS OF THE EUROPEAN-ACADEMY-OF-PAEDIATRIC-SOCIETIES (EAPS); COPENHAGEN, DENMARK; OCTOBER 23 -26, 2010
• A GAVILANES: "Neonatal electrocortical brain activity and cerebral tissue oxygenation during non-acidotic, normocarbic and normotensive graded hypoxemia", CLINICAL NEUROPHYSIOLOGY, vol. 115, no. 2, 1 February 2004 (2004-02-01), pages 282-288, XP055070361, ISSN: 1388-2457, DOI: 10.1016/S1388-2457(03)00336-5

EP 2 836 121 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to methods and apparatus for estimating blood gas tension. More particularly, the present invention relates to methods and apparatus for estimating carbon dioxide and/or oxygen blood tension of premature babies.

Measurement of oxygen and carbon dioxide tension in blood, that is the partial pressure of oxygen and carbon dioxide in blood, is useful in many clinical settings. For example, the tension of oxygen and/or carbon dioxide in the blood of a person can be useful for evaluating whether the person has problems with breathing, or for determining whether ventilation of the person is working correctly.

Preterm babies often have difficulty breathing without assistance and are ventilated to ensure that there is sufficient gas exchange. Intensive care management of preterm babies aims to ensure good oxygen and blood supply to tissues around the body, and in particular to the brain. It is known that carbon dioxide tension in the blood of a preterm baby can affect blood flow to the brain of the preterm baby. Oxygen and carbon dioxide tension in the blood are therefore often monitored in preterm babies.

Oxygen and carbon dioxide tension in the blood of a preterm baby are typically measured by taking a sample of blood from the baby. It is not desirable to take blood from a preterm baby too often and as such a blood sample is typically taken and tested every four hours. Whilst such tests on blood samples provide an accurate indication of the oxygen and carbon dioxide tension in the blood of a preterm baby, it is desirable to be able to constantly monitor oxygen and carbon dioxide tension in the blood of a preterm baby.

Another method of continuously monitoring oxygen and carbon dioxide tension in the blood is by transcutaneous blood gas monitoring. However transcutaneous blood gas monitoring requires skin heating and therefore repeated changes of sensor location is required to avoid skin damage in preterm babies which complicates the use of transcutaneous blood gas monitoring in such subjects.

End-tidal carbon dioxide, indicating the level of carbon dioxide released at the end of expiration, has previously been monitored in preterm babies, however the results from studies using end tidal carbon dioxide monitoring are conflicting, with some studies reporting good correlations with blood carbon dioxide tension and others reporting clinically unacceptable underestimation of blood carbon dioxide tension with poor trending over time.

Improved methods of determining the tension of oxygen and carbon dioxide in blood are therefore desirable.

It is an object of the invention to provide improvements in systems and methods for determining the tension of oxygen or carbon dioxide in blood.

According to a first aspect of the invention there is provided a computer-implemented method of generating data indicating tension of a carbon dioxide or oxygen in blood of a preterm baby based upon data indicating brain activity of said preterm baby. The method comprises receiving said data indicating brain electrical activity of said preterm baby; processing said data indicating brain electrical activity of said preterm baby to generate activity data indicating length of at least one period of relatively low brain activity; processing said data indicating brain electrical activity of said preterm baby to generate power data indicating power of a frequency band of said data indicating brain activity of said preterm baby; and combining said activity data and said power data to generate said data predicting the tension of said carbon dioxide or oxygen in the blood of said preterm baby.

By combining data in this way, it has been found that an accurate indication of tension of the blood gas oxygen or carbon dioxide, in blood of a preterm baby can be generated. This realisation builds on the inventors previous work published in "Effect of carbon dioxide on background cerebral activity and fractional oxygen extraction in very low birth weight infants just after birth", Victor et. al., Pediatric Research, Vol. 58, No. 3, 2005, in which relationships between various EEG data metrics and blood carbon dioxide tension in preterm babies during the first three days after birth using one hour EEG recordings are described. Victor et. al. concluded that lower levels of blood carbon dioxide tension are associated with slowing of EEG and increased cerebral fractional oxygen extraction but did not realise the significant improvements in blood oxygen or carbon dioxide tension estimation provided by the combination of data of the present invention. That is, combining data as claimed provides improved gas tension estimation.

The term preterm baby is intended to indicate a baby that is born at less than 37 weeks gestational age.

The method may further comprise selecting an activity data point of said activity data, said data point having an associated time and selecting a power data point of said power data having an associated time closest to said activity data point. Combining said activity data and said power data may comprise processing said selected activity data point in combination with said selected power data point.

Combining said activity data and said power data may comprise applying a first weight to said selected activity data point and applying a second weight to said selected power data point.

The frequency band may be a frequency band in the range 0Hz to 4Hz. That is, the frequency band may be the delta brainwave frequency band.

The power data may indicate power of said frequency band relative to power of all frequency bands. Processing said data indicating brain electrical activity of said preterm baby to generate power data may comprise selecting a subset of said data indicating brain electrical activity of said preterm baby, and generating power data based upon said subset of said data. The subset may be selected such that the brain electrical activity is relatively stationary, that is the frequency and amplitude components of the signal resulting from the brain electrical activity remain relatively unchanged in the subset of the data.. The subset may be data indicating brain electrical activity of said preterm baby during a predetermined time period, for example the subset may be a contiguous subset of data points in a predetermined time period. The predetermined time period may be approximately two seconds, for example in the range one second to three seconds.

In the Victor et. al. paper described above the relative power of delta EEG was generated using a 10-second window. The inventors have realised that using a 10-second window increases the likelihood of the EEG waveform being non-stationary and violating one of the key assumptions of the Fourier Transformation. The inventors have found that a 2-second window provides a sufficiently small frequency resolution for neonatal EEGs that are generally sampled at a low rate while also increasing the probability that the chosen section of EEG data will be relatively stationary. Furthermore, the relative power of delta EEG band is not normally distributed when short periods of EEG data are analysed-therefore the same regression model is not applicable for continuous monitoring.

The activity data may indicate at least one interburst period, that is a period of relatively low brain activity.

The method may further comprise processing said received data indicating brain electrical activity of said preterm baby to filter artefacts. In the Victor et. al. paper described above a 90th centile of interburst intervals from an hour of EEG recording was used such that no artefact filtering was required. However by filtering artefacts the inventors have realised that individual interburst periods can be used with confidence which allows continuous monitoring of gas tension.

Processing said received data indicating brain electrical activity of said preterm baby to remove artefacts may comprise processing said data indicating brain electrical activity of said preterm baby to generate burst EEG data indicating length of at least one period of relatively high brain electrical activity; and filtering data from the received data based upon a relationship between said burst EEG data and said activity data. Processing said received data indicating brain activity of said preterm baby to remove artefacts may comprise processing said data indicating brain electrical activity of said preterm baby to identify data outside of the normal range of the energy of the brain activity. For example, the artefact removal may be based upon a threshold value that is determined by analysis of a training set of data that is manually marked up by an expert. By automatically processing the brain electrical activity to remove artefacts, improved artefact removal is achieved.

The data indicating brain electrical activity of said preterm baby may be electroencephalography data.

The invention can be implemented in any convenient form. For example computer programs may be provided to carry out the methods described herein. Such computer programs may be carried on appropriate computer readable media which term includes appropriate non-transient tangible storage devices (e.g. discs). Aspects of the invention can also be implemented by way of appropriately programmed computers and other apparatus.

Embodiments of the invention will now be described, by way of example, with reference to the accompanying drawings in which:

Figure 1 is a schematic illustration of a system for generating data indicating tension of carbon dioxide and/or oxygen in the blood of a preterm baby in accordance with the invention;

Figure 1A is a schematic illustration of a computer of the system of Figure 1;

Figure 2 is a flowchart showing processing to determine tension of carbon dioxide and/or oxygen in the blood of a subject in accordance with the invention;

Figure 3 is a flowchart showing part of the processing of Figure 2 in more detail;

Figure 4A is a flowchart showing processing to generate interburst data suitable for use in the processing of Figure 2;

Figure 4B is a representation of EEG data and illustrates generation of interburst data;

Figure 5 is a flowchart showing processing to generate relative power brain activity data;

Figure 6 is a flowchart showing processing to pre-process data;

Figure 7 is a representation of EEG data and illustrates pre-processing of data;

Figure 8 shows data generated according to the invention;

Figure 9 illustrates data generated according to the invention; and

Figure 10 illustrates data generated according to the invention and measured data.

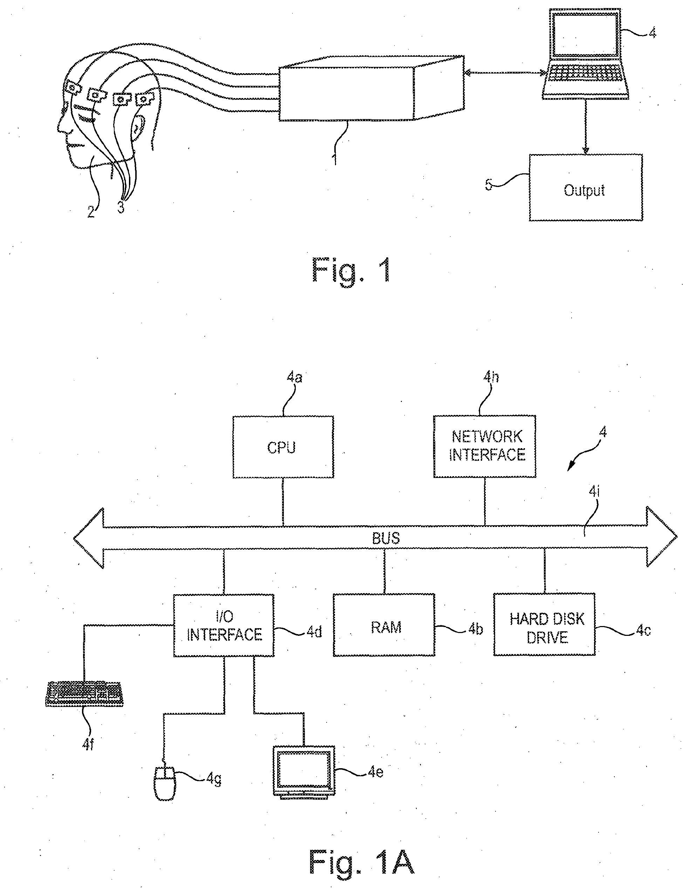

Referring to Figure 1, an Electroencephalography (EEG) apparatus 1 is arranged to measure electrical activity along the scalp of a preterm baby 2 using electrodes 3 placed on the scalp of the preterm baby 2. Typically nine electrodes are used which are placed on the scalp in accordance with the international 10/20 System, although other numbers of electrodes could be used although it has been found that a set of five electrodes, for example at positions C3, C4, O1, O2 and a ground electrode predict a blood carbon dioxide comparable to nine electrodes.

A computer 4 is arranged to receive output data generated by the EEG apparatus 1 indicating the measured electrical activity along the scalp of the premature baby and to process the output data to determine the tension of blood carbon dioxide, measured in kilopascals/ mm Hg, in the blood of the preterm baby 2 and to generate an output 5 indicative of the tension of blood carbon dioxide. For example, the generated output 5 may comprise an alarm signal when the value of the tension of carbon dioxide in blood falls outside of a predetermined range.

Figure 1A shows the computer 4 in further detail. It can be seen that the computer comprises a CPU 4a which is configured to read and execute instructions stored in a volatile memory 4b which takes the form of a random access memory. The volatile memory 4b stores instructions for execution by the CPU 4a and data used by those instructions. For example, in use, the output data generated by the EEG apparatus 1 may be stored in the volatile memory 4b.

The computer 4 further comprises non-volatile storage in the form of a hard disc drive 4c. The output data generated by the EEG apparatus 1 may be stored on the hard disc drive 4c. The computer 4 further comprises an I/O interface 4d to which are connected peripheral devices used in connection with the computer 4. More particularly, a display 4e is configured so as to display output from the computer 4. The display 4e may, for example, display an indication of the tension of blood carbon dioxide. Input devices are also connected to the I/O interface 4d. Such input devices include a keyboard 4f and a mouse 4g, which allow user interaction with the computer 4. A network interface 4h allows the computer 4 to be connected to an appropriate computer network so as to receive and transmit data from and to other computing devices. The CPU 4a, volatile memory 4b, hard disc drive 4c, I/O interface 4d, and network interface 4h, are connected together by a bus 4i.

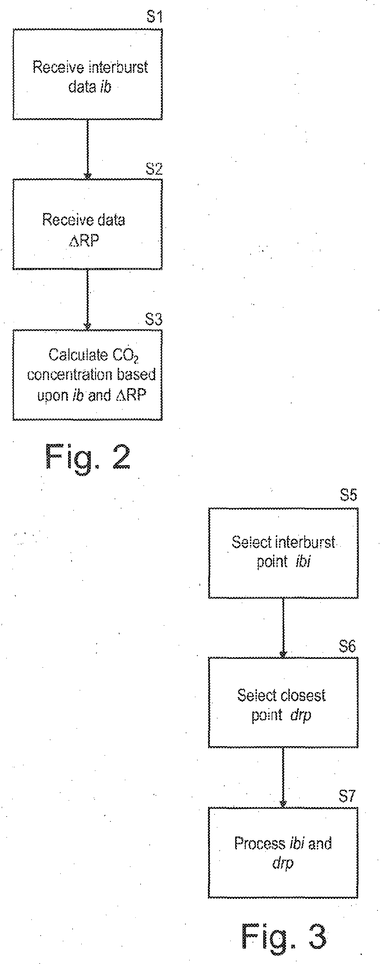

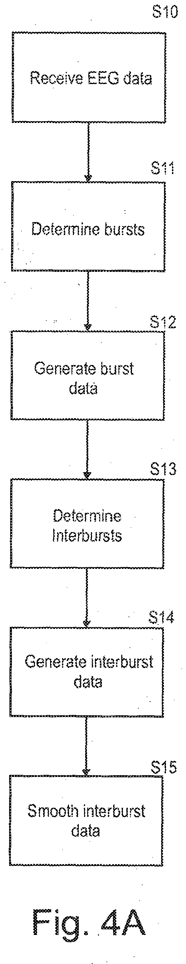

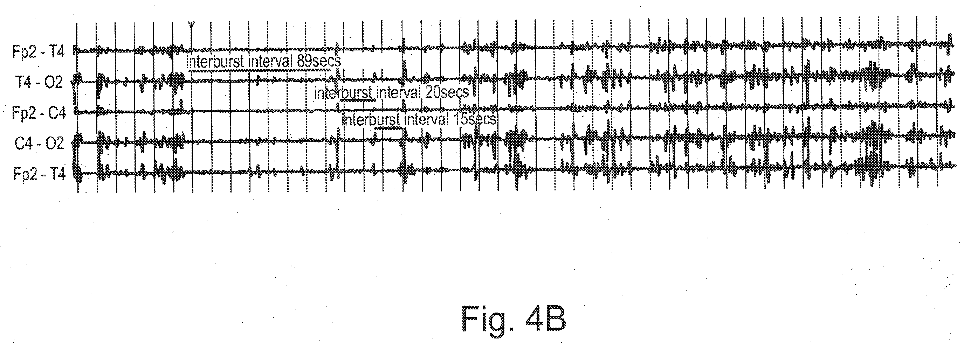

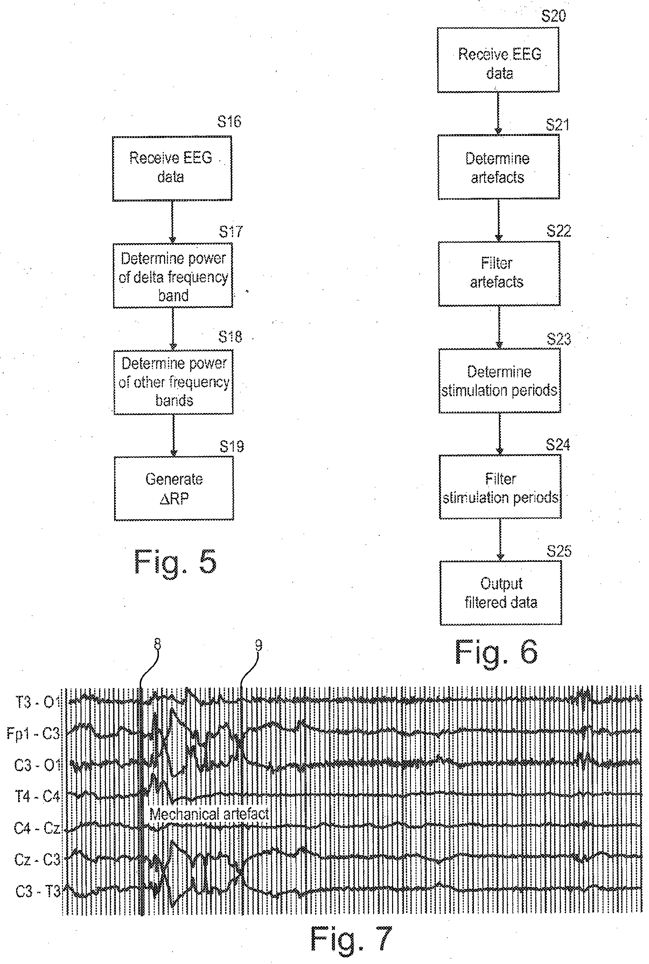

Referring to Figure 2, processing by the computer 4 to determine an indication of the tension of carbon dioxide in the blood of a preterm baby based upon output data generated by the EEG apparatus 1 of Figure 1 is shown. At step S1 activity data interburst interval (*IBI*) is received. The activity data *IBI* indicates duration of periods of relatively low brain activity, sometimes referred to as interburst intervals, of the subject 2. Activity data and the determination thereof is described in detail below with reference to Figures 4A and 4B. At step S2 relative power data RP is received. The relative power data RP is a relative power spectral measure indicating the power of brain waves within a predetermined power range, for example delta brain waves, relative to other waves and is described in detail below with reference to Figure 5. At step S3 tension of blood carbon dioxide is determined based upon a relationship between *IBI* and ΔRP, which is now described with reference to Figure 3.

At step S5 a data point *ibi* is selected from the activity data *IBI* and at step S6 a point *drp* that corresponds to the point *ibi* is selected from the relative power data RP. As described below with reference to Figures 4A and 5, points in the data *IBI* and points in the data RP are each generated from time domain EEG data, typically with data normalisation as described below, and the point *drp* that corresponds to the point *ibi* is the point in the data RP having an associated time closest to a time associated with the point *ibi.*

At step S7 the points *ibi* and *drp* are processed to generate a predicted value for the tension of blood carbon dioxide ($pCO_2$) according to equation (1):

$$pCO_2 = m + a(ibi) + b(drp) \qquad (1)$$

where:

m is a constant;
*a* is a constant weight applied to the value *ibi;* and
*b is* a constant weight applied to the value *drp.*

Suitable values for *m, a and b* have been found to be 6.320, 0.287 and -1.111 respectively, although it will be appreciated that other values will provide suitable results. For example, values in the range 1.938 < *m* < 10.702, 0.003 < *a* < 0.571 and -2.174 < *b* -0.048. The values indicated above have been determined based upon analysis of measured $pCO_2$ and *ibi* and *drp* from a data generated from a plurality of preterm babies and it will be appreciated that other suitable values may be determined in a corresponding way. Similar ranges of values have been found for a reduced set of electrodes.

The data *IBI* and $\Delta$RP are generated from EEG output data which is processed to generate EEG data through signal processing methods.

For example, the data *IBI* and $\Delta$RP may be generated by placing nine electrodes on the scalp of the infant in accordance with the International 10-20 System. Each EEG recording contains 9 separate cerebral signals that includes the fronto polar (Fp1,Fp2), occipital (01,02), temporal (T3, T4), and central (Cz, C3, C4) areas. The nine electrode signals are arranged into a standard 12 channel observation pattern. These channels included the following differential signals: Fp2-T4, T4-O2, Fp2-C4, C4-02, Fp1-T3, T3-O1, Fp1-C3, C3-O1, T4-C4, C4-Cz, Cz-C3 and C3-T3.

Alternatively, a subset of electrodes can be chosen from the full set of nine electrodes, for example to reduce the time of electrode application to the scalp of the infant. For example, a frontal set consisting of five electrodes could include channels Fp2-C4, Fp1-C3 and Cz as the ground. The channels comprise time synchronized data upon which all EEG feature calculations are performed.

The received channel data is split into two separate processing branches and each branch carries the same signal data to be filtered independently. One branch is filtered using a 0.4Hz 4$^{th}$ order Butterworth High Pass Infinite Impulse Response (IIR) filter followed by a 30Hz 6$^{th}$ order Butterworth Low Pass IIR filter. This filtered signal is used to calculate the data *IBI*. The second branch is used for artefact detection and is filtered using a 4$^{th}$ order Butterworth IIR band stop filter with cut off frequencies of 49 - 51Hz, suppressing any 50Hz electrical noise. The resulting signal may be further pre-processed to remove artefacts as described below.

Referring to Figure 4A, processing to generate the data *IBI* is shown. At step S10 the possibly pre-processed EEG data described above is received. The EEG data may additionally be pre-processed as described below with reference to Figure 6. At step S11 the data is processed to identify bursts of activity in the data. A burst is identified from the EEG data by identifying time periods in the data during which the non-linear energy of the signal in any one of the channels exceeds a predetermined threshold, where the non-linear energy of the signal for a channel $a$, $E_a(n)$ is defined as $E_a(n) = x_a(n)x_a(n-3) - x_a(n-1)x_a(n-2)$, where $x_a(n)$ is the signal value on channel a at point n. That is, a moving average over a window length of three data points is used to determine the non-linear energy $E_a(n)$. The value of $E_a(n)$ is further smoothed using a moving summation window that sums every sample value of $E_a(n)$ over a one second period. This process will delay the energy value of the signal by half a second in real time. However, such delays are insignificant given the smoothing of the IBI calculation that takes place.

The predetermined burst threshold used to identify bursts in the data may be determined in any convenient way, for example by analysis of data marked up by an expert to indicate bursts and will typically vary depending on how many electrodes are used in the system. For a 9 electrode (12 channel) system, a burst threshold value of 5932 has been found to be optical, although it will be appreciated that other burst threshold values may be used. Interruptions of relatively short duration, for example a duration of less than one second, that occur during relatively long periods of activity or inactivity, are ignored such that the relatively long period is not divided by the relatively short period. The effect of an interburst being split in two by an artefact is more pronounced the longer the interburst interval. For example an interburst period of 60 seconds would be split into two periods of 30 seconds if a short burst were to be left unsuppressed, whereas a four second burst split into two equal parts would produce two two second bursts. The longer interburst would have a much greater impact on the equation and decrease the predicted $CO_2$ value.

At step S12 the bursts are processed to generate burst data. For each burst identified at step S11 a point b is generated having a time value corresponding to the end point of the burst and an associated value indicating the length of time of the burst.

At step S13 interburst intervals are identified by determining time periods in the data during which no channel exceeds the predetermined threshold. It will be appreciated therefore that each time point in the data is either a burst or an interburst and that interburst periods fall between bursts of activity. At step S14 interburst data is generated in a manner generally corresponding to the generation of burst data. That is, for each interburst interval a data point *ibi* is generated having a time value corresponding to the end point of the interburst interval and an associated value based upon the length of the interburst interval. As illustrated below with reference to Figure 7, the burst data and the interburst data *IBI* can be plotted based upon the time and value associated with each point b and ibi respectively. The interburst data and burst data may be smoothed based upon the plot at step S15 based upon a moving average of the data. The moving average is determined by taking a predetermined number of data points either side of a point *ibi* and taking the average of all these points. A typical smoothing average window size is 51 points *ibi,* which incurs a delay of 25 *ibi* time periods.

Figure 4B shows five channels of the EEG data generated by processing the EEG output data output from the EEG apparatus. In Figure 4B three interburst intervals are indicated, each interburst interval occurring on a different one of the channels, although it will be appreciated that the EEG data of Figure 4B includes additional interburst intervals not indicated. Each interburst interval is indicated on a channel of the five channels shown, however it will be appreciated from the description above that each interburst interval indicates a time period in which the EEG signal of the EEG data does not exceed a predetermined threshold on any one of the channels of the EEG data, including any channels not shown.

Referring to Figure 5, processing to generate relative power data RP is shown. At step S16 EEG data is received. The EEG data received at step S15 corresponds to the EEG data received at step S10 of Figure 4A. At step S17 the power of the selected frequency band of the EEG data, typically the delta frequency band, is determined. The power of the selected frequency band of the EEG data is determined by transforming the EEG data from the time domain into the frequency domain using a Fast Fourier Transformation and determining the power of the data between predetermined frequencies for a two second window around each point such that the window overlaps for a second for each consecutive point. For example where the delta band is used, the power of the data between 0.5Hz and 3.5Hz is determined in a two second window around each point. At step S18 the total power of all frequency bands of the EEG data is determined for each point from the frequency domain data and at step S19 the relative power data RP is determined by dividing the power of the EEG frequency band determined at step S 17 by the power of all frequency bands for each point to generate a value *drp* for each time point of the EEG data.

As described above, the data *IBI* and data RP are processed to determine values of $pCO_2$ providing an estimate of the tension of carbon dioxide in the blood of the preterm baby from whom the EEG data is acquired.

As indicated above, the EEG data may be pre-processed to remove artefacts and pre-processing to remove artefacts will now be described with reference to Figure 6. At step S20 EEG data corresponding to the data resulting from processing the EEG output data is received. At step S21 the EEG data is processed to determine parts of the data that are due to artefacts of the EEG generation process. Artefacts may be caused for example by external contact with the electrodes 3 which may cause an electrical signal to be detected by the EEG apparatus 1 that is not due to electrical activity of the brain of the baby 2 and are detected by determining parts of the EEG data that fall outside of the normal range of detected energy threshold values.

A change in the energy of a signal may be detected by a Non-Linear Energy Operator (NLEO) method. A *primary* NLEO equation proposed is

$$E(n) = x(n)^2 - x(n+1)x(n-1) = A^2 sin^2(\Omega) \approx A^2 \Omega^2$$

Where *x(n)* is the current ($n^{th}$) sample of the signal, $\Omega$ is the frequency in radians $s^{-1}$ and *A* is the amplitude. The second part of the equation highlights that the NLEO value is dependent both on the amplitude and the frequency of the signal.

An updated version of the NLEO spreads the dependence of the NLEO value across an additional signal sample. This version of the NLEO is less dependent on the current sample and hence less prone to changes in noise:

$$E(n) = x(n-l)x(n-p) - x(n-q)x(n-s)$$

For purposes of attempting artefact measurement embodiments of the invention may use the values *l* = 0, *p* = 3, *q* = 1 and *s* = 2, which results in the following *secondary* NLEO equation:

$$E(n) = x(n)x(n-3) - x(n-1)x(n-2)$$

The secondary NLEO equation is more sensitive to changes in increasing frequencies than the primary NLEO method. The secondary NLEO equation possesses a natural notch filter at close to half the Nyquist frequency of the signal.

An artefact occurrence was estimated using the primary NLEO method. A sliding square window is convolved with the NLEO calculations (E) to ensure that the NLEO values transitioned more gradually between values with respect to time. A large number of artefacts have been found to have a majority of the power close to and greater than 1 Hz. Hence, a window size that captured the change of the signal over one second periods is considered to be acceptable. A square window of size *N* = 203 samples, which included the 3 sample signal advancement produced by the NLEO equation, was used throughout all NLEO calculations. A summation of the NLEO values over the window length creates a smoothed NLEO value for each second of data. The calculation, performed every sample, is shown below:

$$E(k)_{smoothed} = \sum_{n=k}^{N+n-1} |E(n)|$$

Artefact detection may be performed on a channel by channel basis. That is, if a single channel exceeded a set threshold, then that section of signal was tagged as artefact. The marking of artefacts on individual channels may lead to the reasonable assumption that signals on all other channels, regardless of their features, were also induced by a non-cerebral source. Hence, all channels may be be marked as artefact and excluded from further signal analysis.

In embodiments of the invention a number of method of artefact detection may be used, including:

- The use of an amplitude threshold detector. The amplitude detector may be arranged such that if the maximum absolute value of any EEG channel exceeded a set threshold, then the segment of the signal was marked as artefact.

$$A = (\max(|eegChannels|) > threshold)$$

- The use of the smoothed secondary NLEO equation. One embodiments use a bandwidth setting of the range normally associated with EEG bursts, 0.1 - 15 Hz. Another embodiment uses the whole bandwidth of the EEG signal, 0 - 100 Hz. A 50 Hz notch filter may be employed for the full bandwidth EEG setting to remove any constant artefact caused by a high electrode contact impedance. This artefact manifested itself as a constant 50 Hz signal that affected the overall artefact detection.

The maximum smoothed NLEO value across all channels may be compared against a threshold. Exceeding the threshold acknowledged the presence of an artefact.

- The use of the primary smoothed NLEO equation with a 50 Hz notch filter. Both the primary and secondary NLEO methods used can be summarised with the following equation:

$$A = (\max(E(k)_{smoothed}) > threshold)$$

It has been found that the narrowband method (0.1-15Hz) may predict an artefact with 98% correct probability. However the wide band primary NLEO method was considered, on average, to provide the highest correlation when compared with manual markings of artefacts by clinicians.

At step S22 the parts of the data that are determined to be due to artefacts of the EEG generation process are filtered from the EEG data by pausing the processing of the data until artefacts are no longer detected in the data, at which point processing recommences.

At step S23 the data resulting from the processing of steps S21 and S22 is processed to determine parts of the data that indicate that the baby 2 is being stimulated at a time indicated by that part of the data. Periods during which the baby is stimulated are determined by identifying time points in the data during which brain activity is relatively high and can be determined based upon the ratio of value indicating length of burst of a point b of the burst data to value indicating length of interburst of a point *ibi* of the interburst data determined at steps S12 and S14 of Figure 4A respectively for two corresponding points *band ibi* where two points *band ibi* correspond if they lie closest in time to one another. For example periods at which the ratio *b/ibi* is greater than one may be excluded although the value at which the ratio *b/ibi* will vary depending on the value specified by the burst threshold. That is, if the burst threshold is decreased the ratio will increase due to more bursts being detected. Conversely, if the burst threshold is increased the ratio value will decrease due to the signal being classified as primarily interburst. At step S24 the parts of the data that are determined to indicate that the baby 2 is being stimulated are also filtered from the EEG data by removing the data and at step S25 the filtered data is output.

Figure 7 shows an example of a mechanical artefact in the EEG data that is to be removed. The data between lines 8 and 9 indicates a mechanical artefact occurring between times associated with the lines, which is to be filtered. The data after filtering is treated as a continuous dataset.

That is, where data associated with a measurement time period is removed from the data at step S22 or S24, data at a time immediately preceding the time period is removed and immediately following the time period that is removed is treated as occurring at consecutive time periods. Therefore, artefact data creates a pause in the processing of the EEG and prediction of blood carbon dioxide tension.

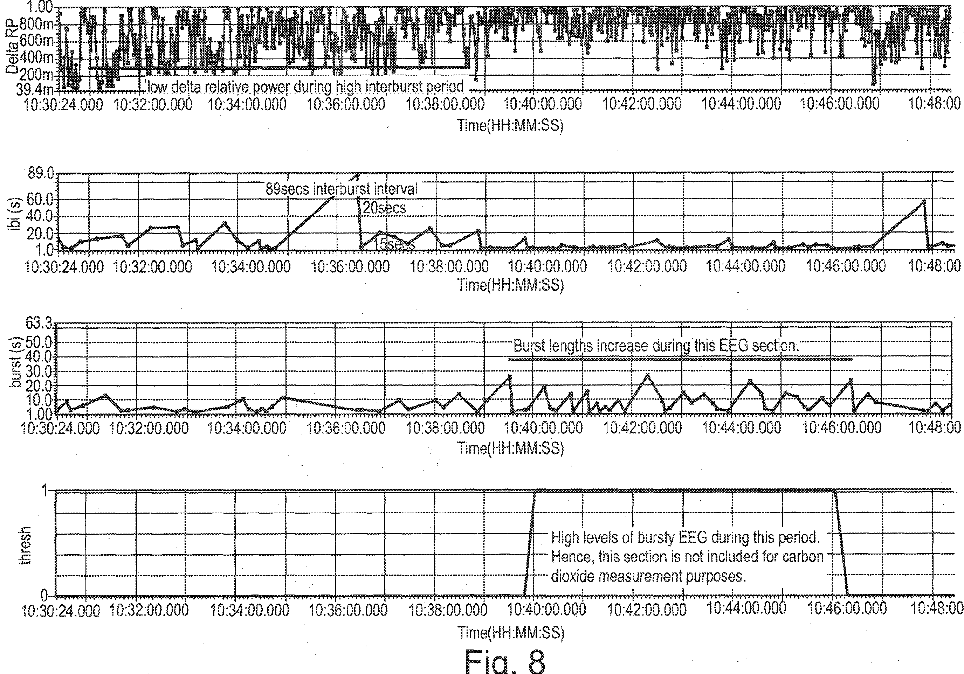

Figure 8 shows data generated according to the processing of Figures 4A and 5. A first trace labelled "Delta RP" indicates the data RP, based upon the delta frequency band, during a time period between 10:30:24 and 10:48:00 and a second trace labelled "ibi (s)" indicates the data *IBI* during the same time period. It can be seen that the values RP fluctuate considerably, however a pattern of relatively low values for points *drp* can be seen during a corresponding period of relatively long interburst periods which illustrates a generally inverse relationship between burst length and relative power.

The relationship and its significance with regard to prediction of blood carbon dioxide tension is reiterated in equation 1. In particular, the relationship between *ibi* and *drp* can be explained as follows. A reduction in carbon dioxide to the brain has the effect of decreasing the observable amplitude of the EEG signal such that interburst length generally increases, and an increased interburst length therefore correlates to an increased amount of cerebral $CO_2$. When observable EEG activity is occurring, indicating an increase in carbon dioxide to the brain and causing generally lower interburst length, the signal possesses predominantly low frequency, high amplitude components with the higher frequency components possessing much smaller amplitudes. That is values of *drp,* indicating the ratio of low frequency band relative to all other frequency bands, will increase. As the signal enters into an interburst interval period, where the signal is flat, the signals begin to reduce in amplitude. This reduction in amplitude is visible in the lower frequency components whereas the higher frequency components of the EEG remain present. Therefore, as the signal becomes visibly flat, the proportion of the low frequency signals to high frequency signals is increased dramatically.

A third trace labelled "burst (s)" indicates length of bursts in the data during the same time period corresponding to the burst data determined at step S12 of Figure 4A. A time period beginning at a time 10:40:00 and lasting until around 10:46:00 can be seen in the data during which a large number of bursts of relatively long length can be seen to occur and during which interburst periods are relatively short. As described above such a period of high activity is indicative of a period of stimulation of the baby and as such is filtered from the data. A fourth trace labelled "thresh" indicates whether a threshold of burst length to interburst length has been exceeded and it can be seen that during the period of high activity the threshold is exceeded such that the EEG data between 10:40:00 and 10:46:00 is removed from the data and no blood carbon dioxide tension estimation is carried out during this time period.

Typically it is desirable to continuously monitor the tension of blood carbon dioxide. As such, typically the above processing is carried out on EEG data that is continuously generated from a baby. For example, the processing described above may be carried out on EEG data that is continuously received from a baby. It will be appreciated that in order to generate interburst data used in the estimation of blood carbon dioxide tension a set of data over a time period is required and as such the processing described above may be carried out at relatively regular intervals, for example each time a data point *ibi* is determined as described above with reference to Figure 4A.

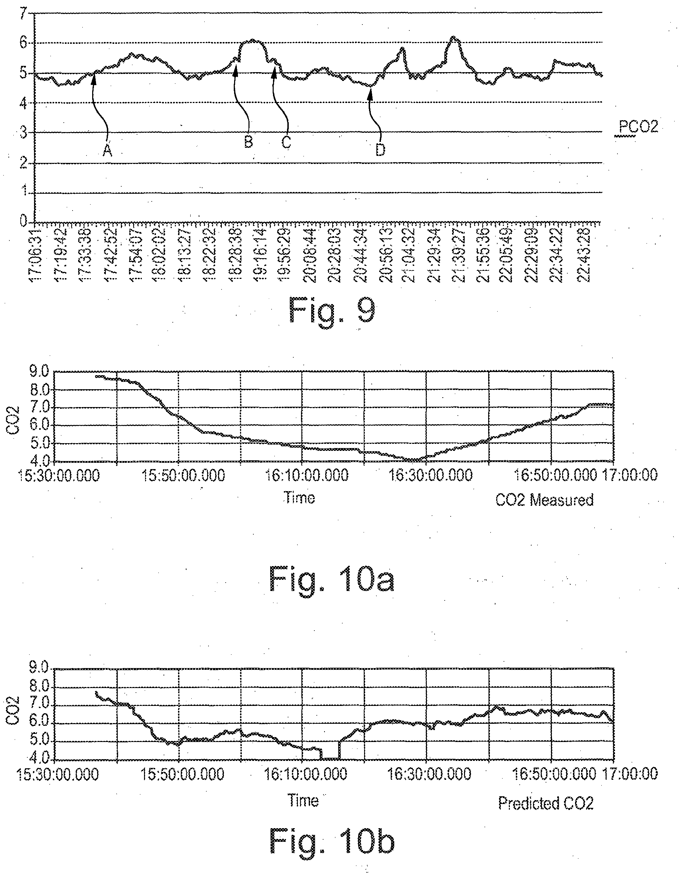

Figure 9 illustrates predicted $CO_2$ according to the invention ($PCO_2$) in kPa in a baby born at 27 weeks' gestation with normal cranial ultrasound scan. The EEG recording was commenced at a time of 17:06. A clinician performed a capillary blood gas at 17.37 (event A) which showed that measured blood $CO_2$ ($pCO_2$) was 5.25 kPa. The corresponding $PCO_2$ at 17.37 using EEG was determined to be 5 kPa. The baby was then extubated (taken off the ventilator) at 18.40 (event B). A rise in $PCO_2$ is recorded following extubation. The clinician returns at 19.54 to repeat a capillary blood gas (event C) which shows that the $pCO_2$ was 5.23 kPa. The corresponding $PCO_2$ at 19.54 using EEG was determined to be 5.5 kPa. Nursing staff handled the baby for routine baby cares (nappy change etc) at 21.00 hours (event D). A rise in $pCO_2$ is observed during this period when cares was performed. The graph shows that $PCO_2$ is within 0.3 kPa accuracy of blood $pCO_2$. It also shows that the trends/ changes in $PCO_2$ are consistent with what is expected in relation to clinical events.

Where an observed offset is recorded between $pCO_2$ and $PCO_2$ embodiments of the invention may include an offset value which may be input by a user and applied to the determined $PCO_2$ to correct toward the measured value of $pCO_2$ since the offset may be substantially constant for all values of determined $PCO_2$.

Figure 10 illustrates a comparison between simultaneous transcutaneous carbon dioxide monitoring and predicted carbon dioxide monitoring ($PCO_2$) using features of neonatal EEG. Figure 10 illustrates a section of continuous prediction of carbon dioxide using features of neonatal EEG. This predicted $PCO_2$ shows strong correlation with simultaneously measured transcutaneous carbon dioxide measurements. Recordings were commenced at 15.30 and were carried on for 1.5 hours. The trend in transcutaneous carbon dioxide has been shown in Figure 10a. Simultaneous trend in $PCO_2$ over the same time is shown in Figure 10b.

Although specific embodiments of the invention have been described above, it will be appreciated that various modifications can be made to the described embodiments without departing from the scope of the present invention. That is, the described embodiments are to be considered in all respects exemplary and non-limiting. In particular, where a particular form has been described for particular processing, it will be appreciated that such processing may be carried out in any suitable form arranged to provide suitable output data.

**Claims**

**1.** A computer-implemented method of generating data indicating tension of oxygen and/ or carbon dioxide in blood of a preterm baby based upon data indicating brain activity of said preterm baby, the method comprising:

receiving said data indicating brain electrical activity of said preterm baby;
processing said data indicating brain electrical activity of said preterm baby to generate activity data indicating length of at least one period of relatively low brain activity;

processing said data indicating brain electrical activity of said preterm baby to generate power data indicating power of a frequency band of said data indicating brain activity of said preterm baby; and

combining said activity data and said power data to generate said data predicting the tension of oxygen and/or carbon dioxide in the blood of said preterm baby.

2. A computer-implemented method according to claim 1, further comprising:

selecting an activity data point of said activity data, said data point having an associated time;

selecting a power data point of said power data having an associated time closest to said activity data point; and

wherein combining said activity data and said power data comprises processing said selected activity data point in combination with said selected power data point.

3. A computer-implemented method according to claim 2, wherein combining said activity data and said power data comprises applying a first weight to said selected activity data point and applying a second weight to said selected power data point.

4. A computer-implemented method according to any preceding claim, wherein said frequency band is a frequency band in the range 0Hz to 4Hz.

5. A computer-implemented method according to any preceding claim, wherein said power data indicates power of said frequency band relative to power of all frequency bands.

6. A computer-implemented method according to any preceding claim, wherein processing said data indicating brain electrical activity of said preterm baby to generate power data comprises:

selecting a subset of said data indicating brain electrical activity of said preterm baby, and generating power data based upon said subset of said data.

7. A computer-implemented method according to claim 6, wherein said subset is selected such that the brain electrical activity is relatively stationary.

8. A computer-implemented method according to claim 6 or 7, wherein said subset is data indicating brain electrical activity of said preterm baby during a predetermined time period; optionally said predetermined time period is about two seconds.

9. A computer-implemented method according to any preceding claim, wherein said activity data indicates at least one interburst period.

10. A computer-implemented method according to any preceding claim, further comprising:

processing said received data indicating brain electrical activity of said preterm baby to filter artefacts.

11. A computer-implemented method according to claim 7, wherein processing said received data indicating brain electrical activity of said preterm baby to remove artefacts comprises:

processing said data indicating brain electrical activity of said preterm baby to generate burst EEG data indicating length of at least one period of relatively high brain electrical activity; and

filtering data from the received data based upon a relationship between said burst EEG data and said activity data.

12. A computer-implemented method according to claim 10 or 11, wherein processing said received data indicating brain activity of said preterm baby to remove artefacts comprises:

processing said data indicating brain electrical activity of said preterm baby to identify data outside of the normal range of the energy of the brain activity.

13. A computer-implemented method according to any preceding claim, wherein said data indicating brain electrical activity of said preterm baby is electroencephalography data.

14. A computer program comprising computer readable instructions configured to cause a computer to carry out a method according to any preceding claim; optionally the computer program being carried on a computer readable medium.

15. A computer apparatus generating data indicating tension of oxygen or carbon dioxide in blood of a preterm baby based upon data indicating brain activity of said preterm baby, the computer apparatus comprising:

> a memory storing processor readable instructions; and
> a processor arranged to read and execute instructions stored in said memory;

wherein said processor readable instructions comprise instructions arranged to control the processor to carry out the method of any one of claims 1 to 13.

**Patentansprüche**

1. Computer-implementiertes Verfahren zur Erzeugung von Daten zur Anzeige des Drucks von Sauerstoff und/oder Kohlendioxid im Blut eines Frühgeborenen basierend auf Daten zur Anzeige der Hirnaktivität des Frühgeborenen, das Verfahren umfassend:

> den Empfang der Daten zur Anzeige der elektrischen Hirnaktivität des Frühgeborenen;
> die Verarbeitung der Daten zur Anzeige der elektrischen Hirnaktivität des Frühgeborenen zur Erzeugung von Aktivitätsdaten zur Anzeige der Länge von wenigstens einem Zeitraum mit relativ niedriger Hirnaktivität;
> die Verarbeitung der Daten zur Anzeige der elektrischen Hirnaktivität des Frühgeborenen zur Erzeugung von Leistungsdaten zur Anzeige der Leistung eines Frequenzbands der Daten zur Anzeige der elektrischen Hirnaktivität des Frühgeborenen und
> die Kombination der Aktivitätsdaten und der Leistungsdaten zur Erzeugung der Daten zur Prognose des Drucks von Sauerstoff und/oder Kohlendioxid im Blut des Frühgeborenen.

2. Computer-implementiertes Verfahren nach Anspruch 1, ferner umfassend:

> die Auswahl eines Aktivitätsdatenpunkts der Aktivitätsdaten, wobei der Datenpunkt eine zugehörige Zeit hat;
> die Auswahl eines Leistungsdatenpunkts der Leistungsdaten mit einer zugehörigen Zeit, die am nächsten an dem Aktivitätsdatenpunkt liegt; und
> wobei die Kombination der Aktivitätsdaten und der Leistungsdaten die Verarbeitung des ausgewählten Aktivitätsdatenpunkts in Verbindung mit dem ausgewählten Leistungsdatenpunkt umfasst.

3. Computer-implementiertes Verfahren nach Anspruch 2, wobei die Kombination der Aktivitätsdaten und der Leistungsdaten die Anwendung eines ersten Gewichts auf den ausgewählten Aktivitätsdatenpunkt und die Anwendung eines zweiten Gewichts auf den ausgewählten Leistungsdatenpunkt umfasst.

4. Computer-implementiertes Verfahren nach einem der vorhergehenden Ansprüche, wobei das Frequenzband ein Frequenzband im Bereich von 0 Hz bis 4 Hz ist.

5. Computer-implementiertes Verfahren nach einem der vorhergehenden Ansprüche, wobei die Leistungsdaten die Leistung des Frequenzbands in Bezug auf die Leistung aller Frequenzbänder anzeigen.

6. Computer-implementiertes Verfahren nach einem der vorhergehenden Ansprüche, wobei die Verarbeitung der Daten zur Anzeige der elektrischen Hirnaktivität des Frühgeborenen zur Erzeugung von Leistungsdaten Folgendes umfasst:

> die Auswahl einer Teilmenge der Daten zur Anzeige der elektrischen Hirnaktivität des Frühgeborenen und die Erzeugung von Leistungsdaten basierend auf der Teilmenge der Daten.

7. Computer-implementiertes Verfahren nach Anspruch 6, wobei die Teilmenge so ausgewählt wird, dass die elektrische Hirnaktivität relativ stationär ist.

8. Computer-implementiertes Verfahren nach Anspruch 6 oder 7, wobei die Teilmenge in Daten zur Anzeige der elektrischen Hirnaktivität des Frühgeborenen in einem vorher festgelegten Zeitraum besteht, wobei optional der vorher festgelegte Zeitraum etwa zwei Sekunden beträgt.

9. Computer-implementiertes Verfahren nach einem der vorhergehenden Ansprüche, wobei die Aktivitätsdaten wenigstens einen Interburst-Zeitraum anzeigen.

10. Computer-implementiertes Verfahren nach einem der vorhergehenden Ansprüche, ferner umfassend:

die Verarbeitung der empfangenen Daten zur Anzeige der elektrischen Hirnaktivität des Frühgeborenen zur Filterung von Artefakten.

11. Computer-implementiertes Verfahren nach Anspruch 7, wobei die Verarbeitung der empfangenen Daten zur Anzeige der elektrischen Hirnaktivität des Frühgeborenen zur Entfernung von Artefakten Folgendes umfasst:

die Verarbeitung der Daten zur Anzeige der elektrischen Hirnaktivität des Frühgeborenen zur Erzeugung von Burst-EEG-Daten zur Anzeige der Länge von wenigstens einem Zeitraum mit relativ hoher elektrischer Hirnaktivität und
die Filterung von Daten aus den empfangenen Daten basierend auf einer Beziehung zwischen den Burst-EEG-Daten und den Aktivitätsdaten.

12. Computer-implementiertes Verfahren nach Anspruch 10 oder 11, wobei die Verarbeitung der empfangenen Daten zur Anzeige der Hirnaktivität des Frühgeborenen zur Entfernung von Artefakten Folgendes umfasst:

die Verarbeitung der Daten zur Anzeige der elektrischen Hirnaktivität des Frühgeborenen zur Identifikation von Daten außerhalb des normalen Bereichs der Energie der Hirnaktivität.

13. Computer-implementiertes Verfahren nach einem der vorhergehenden Ansprüche, wobei die Daten zur Anzeige der elektrischen Hirnaktivität des Frühgeborenen Elektroenzephalographiedaten sind.

14. Computerprogramm, umfassend computerlesbare Anweisungen, die so konfiguriert sind, dass ein Computer zur Ausführung eines Verfahrens nach einem der vorhergehenden Ansprüche veranlasst wird, wobei optional das Computerprogramm auf einem computerlesbaren Medium gespeichert ist.

15. Computervorrichtung zur Erzeugung von Daten zur Anzeige des Drucks von Sauerstoff und/oder Kohlendioxid im Blut eines Frühgeborenen basierend auf Daten zur Anzeige der Hirnaktivität des Frühgeborenen, die Computervorrichtung umfassend:

einen Speicher, auf dem prozessorlesbare Anweisungen gespeichert sind; und
einen Prozessor, der zum Lesen und zur Ausführung von in dem Speicher gespeicherten Anweisungen angeordnet ist, wobei die prozessorlesbaren Anweisungen umfassen, die zur Steuerung des Prozessors zur Ausführung des Verfahrens nach einem der Ansprüche 1 bis 13 angeordnet sind.

**Revendications**

1. Procédé mis en oeuvre par ordinateur de génération de données indiquant une tension en oxygène et/ou en dioxyde de carbone dans le sang d'un bébé prématuré sur la base de données indiquant une activité cérébrale dudit bébé prématuré, le procédé consistant à :

recevoir lesdites données indiquant une activité électrique cérébrale dudit bébé prématuré ;
traiter lesdites données indiquant une activité électrique cérébrale dudit bébé prématuré pour générer des données d'activité indiquant une longueur d'au moins une période d'activité cérébrale relativement faible ;
traiter lesdites données indiquant une activité électrique cérébrale dudit bébé prématuré pour générer des données de puissance indiquant une puissance d'une bande de fréquences desdites données indiquant une activité cérébrale dudit bébé prématuré ; et
combiner lesdites données d'activité et lesdites données de puissance pour générer lesdites données prédictives de la tension en oxygène et/ou en dioxyde de carbone dans le sang dudit bébé prématuré.

**2.** Procédé mis en oeuvre par ordinateur selon la revendication 1, consistant en outre à :

sélectionner un point de données d'activité desdites données d'activité, ledit point de données étant associé à un instant ;
sélectionner un point de données de puissance desdites données de puissance associées à un instant le plus proche dudit point de données d'activité ; et
dans lequel la combinaison desdites données d'activité et desdites données de puissance consiste à traiter ledit point de données d'activité sélectionné en combinaison avec ledit point de données de puissance sélectionné.

**3.** Procédé mis en oeuvre par ordinateur selon la revendication 2, dans lequel la combinaison desdites données d'activité et desdites données de puissance consiste à appliquer une première pondération audit point de données d'activité sélectionné et à appliquer une seconde pondération audit point de données de puissance sélectionné.

**4.** Procédé mis en oeuvre par ordinateur selon l'une quelconque des revendications précédentes, dans lequel ladite bande de fréquences est une bande de fréquences comprise dans la gamme de 0 Hz à 4 Hz.

**5.** Procédé mis en oeuvre par ordinateur selon l'une quelconque des revendications précédentes, dans lequel lesdites données de puissance indiquent la puissance de ladite bande de fréquences par rapport à la puissance de toutes les bandes de fréquences.

**6.** Procédé mis en oeuvre par ordinateur selon l'une quelconque des revendications précédentes, dans lequel le traitement desdites données indiquant une activité électrique cérébrale dudit bébé prématuré de façon à générer des données de puissance consiste à :

sélectionner un sous-ensemble desdites données indiquant une activité électrique cérébrale dudit bébé prématuré, et générer des données de puissance sur la base dudit sous-ensemble desdites données.

**7.** Procédé mis en oeuvre par ordinateur selon la revendication 6, dans lequel ledit sous-ensemble est sélectionné de sorte que l'activité électrique cérébrale soit relativement stationnaire.

**8.** Procédé mis en oeuvre par ordinateur selon la revendication 6 ou 7, dans lequel ledit sous-ensemble est constitué de données indiquant une activité électrique cérébrale dudit bébé prématuré pendant une période de temps prédéfinie, ladite période de temps prédéfinie étant éventuellement d'environ deux secondes.

**9.** Procédé mis en oeuvre par ordinateur selon l'une quelconque des revendications précédentes, dans lequel lesdites données d'activité indiquent au moins une période entre salves.

**10.** Procédé mis en oeuvre par ordinateur selon l'une quelconque des revendications précédentes, consistant en outre à :

traiter lesdites données reçues indiquant une activité électrique cérébrale dudit bébé prématuré de façon à filtrer des artefacts.

**11.** Procédé mis en oeuvre par ordinateur selon la revendication 7, dans lequel le traitement desdites données reçues indiquant une activité électrique cérébrale dudit bébé prématuré de façon à éliminer des artefacts consiste à :

traiter lesdites données indiquant une activité électrique cérébrale dudit bébé prématuré pour générer des données d'EEG en salves indiquant une longueur d'au moins une période d'activité cérébrale relativement élevée ; et
filtrer des données des données reçues sur la base d'une relation entre lesdites données d'EEG en salves et lesdites données d'activité.

**12.** Procédé mis en oeuvre par ordinateur selon la revendication 10 ou 11, dans lequel le traitement desdites données indiquant une activité cérébrale dudit bébé prématuré de façon à éliminer des artefacts consiste à :

traiter lesdites données indiquant une activité électrique cérébrale dudit bébé prématuré pour identifier des données extérieures à la plage normale de l'énergie de l'activité cérébrale.

**13.** Procédé mis en oeuvre par ordinateur selon l'une quelconque des revendications précédentes, dans lequel lesdites données indiquant une activité électrique cérébrale dudit bébé prématuré sont des données d'électroencéphalographie.

**14.** Programme informatique comprenant des instructions lisibles par ordinateur conçues pour amener un ordinateur à exécuter un procédé selon l'une quelconque des revendications précédentes ; le programme informatique étant éventuellement exécuté sur un support lisible par ordinateur.

**15.** Appareil informatique de génération de données indiquant une tension en oxygène et/ou en dioxyde de carbone dans le sang d'un bébé prématuré sur la base de données indiquant une activité cérébrale dudit bébé prématuré, l'appareil informatique comprenant :

une mémoire contenant des instructions lisibles par processeur ; et
un processeur conçu pour lire et exécuter les instructions mémorisées dans ladite mémoire ;

dans lequel lesdites instructions lisibles par processeur comprennent des instructions conçues pour commander le processeur pour qu'il exécute le procédé selon l'une quelconque des revendications 1 à 13.

Fig. 1

Fig. 1A

S1

Receive interburst
data *ib*

S2

Receive data
$\Delta$RP

S3

Calculate $CO_2$
concentration based
upon *ib* and $\Delta$RP

# Fig. 2

S5

Select interburst
point *ibi*

S6

Select closest
point *drp*

S7

Process *ibi* and
*drp*

# Fig. 3

Fig. 4A

Fig. 4B

S16

Receive EEG
data

S17

Determine power
of delta frequency
band

S18

Determine power
of other frequency
bands

S19

Generate
ΔRP

## Fig. 5

S20

Receive EEG
data

S21

Determine
artefacts

S22

Filter
artefacts

S23

Determine
stimulation periods

S24

Filter
stimulation periods

S25

Output
filtered data

## Fig. 6

8          9

T3 - O1

Fp1 - C3

C3 - O1

T4 - C4

Mechanical artefact

C4 - Cz

Cz - C3

C3 - T3

## Fig. 7

Fig. 8

Fig. 9

Fig. 10a

Fig. 10b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VICTOR.** Effect of carbon dioxide on background cerebral activity and fractional oxygen extraction in very low birth weight infants just after birth. *Pediatric Research,* 2005, vol. 58 (3